# EUROPEAN PATENT SPECIFICATION

(11) **EP 2 762 173 B1**
(45) Date of publication and mention of the grant of the patent: **28.10.2020**
(21) Application number: 12835956.9
(22) Date of filing: 27.09.2012
(51) Int. Cl.: A61L 27/04, B22F 3/11, A61L 27/56, C22C 1/08, C22C 27/02, B22F 3/22

(54) **PREPARATION METHOD FOR MEDICAL POROUS TANTALUM IMPLANT MATERIAL**
VERFAHREN ZUR HERSTELLUNG PORÖSER MEDIZINISCHER TANTALIMPLANTATMATERIALIEN
PROCÉDÉ DE PRÉPARATION D'UN MATÉRIAU AU TANTALE POREUX POUR IMPLANT MÉDICAL

(30) Priority: 29.09.2011 CN 201110300340
(43) Date of publication of application: 06.08.2014
(73) Proprietor: Chongqing Runze Pharmaceutical Company Limited, Chongqing 401120 (CN)
(72) Inventor: YE, Lei, Chongqing 401120 (CN)
(74) Representative: Kitzler, Michael
(86) International application number: PCT/CN2012/082220
(87) International publication number: WO 2013/044832

(56) References cited:
- EP-A1- 0 560 279
- WO-A1-02/066693
- WO-A1-02/066693
- WO-A2-2005/095029
- CN-A- 101 405 039
- CN-A- 101 660 076
- US-A- 3 111 396
- US-A1- 2003 153 981
- JIA PING LI ET AL: "Preparation and Characterization of Porous Titanium", KEY ENGINEERING MATERIALS, vol. 218-220, 1 January 2002 (2002-01-01), pages 51-54, XP055201568, DOI: 10.4028/www.scientific.net/KEM.218-220.51
- XIA, FENGJIN ET AL.: 'Preparation Methods of Porous Tantalum' SCIENCE AND TECHNOLOGY INNOVATION HERALD vol. 1, January 2008, pages 83 - 84, XP008173947
- JIE, YUNFENG ET AL.: 'Fabrication of Porous Niobium-Based Biological Materials via Impregnation and Their Properties' RARE METAL MATERIALS AND ENGINEERING vol. 39, no. 11, November 2010, pages 2015 - 2017, XP008173887
- Anonymous: "Shore Durometer Conversion Chart for polymer", , 30 January 2020 (2020-01-30), XP055663580, Retrieved from the Internet: URL:http://polymerdatabase.com/polymer%20p hysics/Shore%20Table.html [retrieved on 2020-01-30]
- "ISO 868:2003 Plastics and ebonite -- Determination of indentation hardness by means of a durometer (Shore hardness)", INTERNATIONAL STANDARD ISO/IEC, XX, XX, vol. ISO 868, 1 January 2003 (2003-01-01), pages 1-5, XP009105028,

## Description

### Field of the Invention

The present invention relates to a preparation method for medical porous metal implant material, especially relates to a preparation method for medical porous tantalum implant material used for substituting a load-bearing bone tissue.

### Description of the Prior art

A medical porous metal implant material has important and specific applications in treating traumatic osseous tissues, necrotic femoral tissues or in substituting the compact bone tissues such as teeth and the like. Such materials are normally porous stainless steel, porous titanium, and so on. As a porous implant material for the treatment of traumatic osseous tissues and necrotic femoral tissues, the porosity thereof should reach 30-80%, and the pores should be all interconnected and well-distributed, or the growth phase thereof should be uniform to that of the bone tissue, and the weight thereof is lowered to suitable for use in medical implantation.

Due to good biocompatibility of the refractory metal tantalum, the porous form thereof is potential to be used in place of the traditional medical metal biomaterials mentioned above. Also, due to the harmlessness, non-toxicity, few of side effects, as well as the further knowledge of tantalum as implant materials along with the rapid development of the domestic and overseas medicine, the requirement of porous tantalum for medical implantation is getting more urgent than before, and the demand for which is getting much higher. Wherein, as a porous tantalum for medical implantation, if it has a lot of well-distributed interconnecting pores as well as physical and mechanical properties adaptable to human body, then it is expected to be a novel equivalent material of bony tissues.

The porous metal materials for medical implantation are manufactured mainly by powder sintering, like the preparation of common porous metal materials. Especially for obtaining porous metals having foam structure with well-distributed and interconnecting pores, a method of powder sintering as impregnating an organic foam body with metal powder slurry, drying and then sintering is always used, which called foam impregnation for short. However, the porous metal materials produced by powder sintering usually do not have sufficient mechanical properties of metal, mainly because the problems on arrangement of the supporting and elimination relationship of the pore-forming media, as well as on the collapse during sintering process of the metal powder. For now, such problems have not been solved according to any known references, and are overlooked.

There are few references on the metal powder sintering process for preparing porous tantalum, barely no references has mentioned the preparation of porous tantalum for medical implantation by porous tantalum powder sintering. CN Application No. CN200510032174 titled "Three-dimensional through-hole or part-hole interconnecting porous metal foam and its preparing method" and CN Application No. CN200710152394 titled "Porous foam tungsten and preparation method thereof" can be referred to. Nevertheless, the porous metal obtained is for the applications as filtering materials, or used in aerospace and other high temperature environments, not as medical metal implant material. Furthermore, the porous metal processed is not porous tantalum.

Regarding porous tantalum, US Patent No. 5282861 discloses open cell tantalum structures for cancellous bone implants and cell and tissue receptors. The porous tantalum is manufactured by commercial pure tantalum and a supporter such as a carbon skeleton obtained from heat degradation of polyurethane precursors. The carbon skeleton has multiple dodecahedrons with reticulated structures inside, pores wholly distributed, and the porosity thereof reaches 98%. Next, the commercial pure tantalum is bound to the carbon skeleton to form porous metal microstructure through chemical vapor deposition and infiltration, which is called chemical deposition for short. The porous tantalum materials obtained by such process has a tantalum layer with 40 - 60µm in thickness, and the weight proportions of tantalum is about 99% and of carbon skeleton is about 1% based on the weight of the whole porous material. The patent further discloses that the porous material has a compressive strength of 50-70MPa, an elastic modulus of 2.5-3.5GPa, a tensile strength of 63MPaand an amount of plastic deformation of 15%. However, when the porous tantalum described above is used as medical implant material for compact bone tissues such as teeth or the like, the mechanical properties such as the ductility thereof is obviously insufficient which will influence the subsequent processing such as cutting of the formed part. Similarly, the product prepared by the metal powder sintering methods mentioned above all has such problems. WO02/066693A1 discloses a process to produce Ti/Ta prosthetic implant using the replica method, wherein the PU/Ti is squeezed repeatedly "until evenly coated". US2003/153981A1 discloses a process to form Ti-foam used as a bone implant using the PU-replica method wherein the impregnation of the slurry onto the PU can be performed by spraying/vibrational technique.

### Summary of the Invention

The objective of the present invention is to provide a method for preparing medical porous tantalum implant material with good biocompatibilities and mechanical properties, and the method is easily performed and suitable for industrial manufacture.

The objective of the invention is achieved through the appended claim 1, namely : a preparation method for medical porous tantalum implant material, comprise mixing a polyvinyl alcohol solution and a tantalum powder to form a tantalum slurry, injecting the tantalum slurry into an organic foam body through pressurization with vibration, and going through steps of drying, degreasing, sintering, cooling and thermal treatment to obtain the medical porous tantalum. The mass concentration of the aqueous solution of polyvinyl alcohol is 2-8% and the frequency of vibration is 20 - 80 times/min. The thermal treatment refers to: under a vacuity of 10⁻⁴ Pa - 10⁻³Pa, increasing the temperature to 800 - 900°C at a rate of 10 - 20 °C/min, then keeping the temperature for 240 - 480 minutes, then decreasing the temperature to 400°C at a rate of 2 - 5 °C/min, and keeping the temperature for 120 - 300 min, then cooling down to room temperature at a rate of 18-23 °C/min.

During a long-term research and development, the inventors have found that the method of pressurization with vibration used in the present invention can effectively avoid the poor dispersity of the tantalum powder added to the foam body which can lead to uneven distribution, and can severely influence the mechanical properties of the porous tantalum material obtained. During the research and development of medical porous metal materials, as an alternative to load-bearing bone tissues, the medical porous metal materials require high porosity for well-binding of human tissues and good biocompatibility, and therefore can sufficiently play their roles. However, the higher porosity and the bigger pore diameter might weaken the mechanical properties such as strength and toughness; otherwise, well mechanical properties could lead to too low porosity, bad biocompatibility and too high density, which can result in discomfort. There are numerous routes for the preparation of medical porous tantalum, but the inventors creatively put forward the above steps and the process for preparing medical porous tantalum implant materials, especially the thermal treatment process mentioned above is used to fully eliminate the internal stress, make the organization of the porous tantalum materials more evenly, greatly improve the toughness of the prepared porous tantalum materials, and make the process quick and easy. After tests, the obtained porous tantalum materials show a impurity content of less than 0.2%, good biocompatibility and bio-safety, a density up to 5.00 - 8.33 g/cm³, a porosity up to 50 - 70%, a pore diameter up to 150 - 500µm; an elastic modulus up to 4.5 - 6.0Gpa, a extension rate of 10.0 - 11.7%, a bending strength up to 90 - 110Mpa, a compressive strength up to 70 - 80Mpa, and the biocompatibility as well as toughness thereof is close to that of human load-bearing bone tissue. The porous tantalum obtained by the invention is the medical implant materials that suitable for replacing the load-bearing bone tissues. Meanwhile, the preparation method mentioned above is simple and easy to control. The entire preparation process is harmless, non-polluting, without toxic dust, having no side effects on the human body, and is suitable for industrial scale production. Furthermore, in the preparation process, reagents and organic foam bodies that can be completely decomposed without residual in the sintering process are preferably used, which are in favor of ensuring the biocompatibility and bio-safety of the implant materials.

The further characteristics of the present disclosure are using a tantalum powder having an average diameter less than 43 µm and an oxygen content less than 0.1%, as well as using a polyvinyl alcohol solution as a binder and water as a dispersant to form the tantalum slurry with the above mentioned tantalum powder. The organic foam body is polyurethane foam. Then remove water by vacuum drying.

Wherein as a further characteristic of the present disclosure, the polyvinyl alcohol is heated to dissolve with distill water. An aqueous solution of polyvinyl alcohol with 4 - 5% mass percentage and a tantalum powder are used to form a tantalum slurry, in which, 6-9 weight proportions (preferably 7 weight proportions) of a tantalum powder is added into 1 weight proportion of the above aqueous solution of polyvinyl alcohol, and they are mixed homogeneously to form a pasty substance. The tantalum slurry is filled into a polyurethane foam body which having a pore diameter of 0.48 - 0.89mm (preferably 0.56 - 0.72mm), a density of 0.015 - 0.035 g/cm³ (preferably 0.025 g/cm³) and a hardness of larger than 50° (preferably 50° - 80°) by pressurization with vibration (a frequency of the vibration is preferably 60 times/min, the pressure is preferably 0.1MPa).

Further preferably, after the above tantalum slurry has been filled into the organic foam body, the aqueous solution of polyvinyl alcohol is uniformly sprayed on the surface of the organic foam body, and the ratio of the weight proportion of the sprayed aqueous solution of polyvinyl alcohol to weight proportion of the tantalum powder mentioned above is 1 : 6.

The further characteristics of another aspect of the present invention are: keeping a vacuity of drying between 10⁻² - 1then in the protective atmosphere such as a vacuity of 10⁻⁴ - 10⁻³Pa and in a temperature of 400 - 800°C, performing degreasing process to remove polyvinyl alcohol and the organic foam body; then performing vacuum sintering under a vacuity of no less than 10⁻⁴ - 10⁻³Pa with a temperature of 2000 - 2200°C, and keeping the temperature for 1 - 5 hours to obtain the porous sintered body. When keeping the temperature in the sintering process, inert gas can be introduced to replace the vacuum protection. At last, the porous sintered body undergoes annealing treatment.

The conditions of the above mentioned degreasing process further comprise: increasing the temperature to 400-800°C by stages at a rate of 0.5 °C/min - 5 °C/min, introducing argon to form protective atmosphere, and keeping the temperature for 30 min - 120 min;
The conditions of vacuum sintering further comprise: under a vacuity of no less than 10⁻³Pa, increasing a temperature from room temperature to 1200°C - 1500°C at a rate of no higher than 10 - 20 °C/min and keeping the temperature for 1 h - 2 h; and then increasing the temperature to 2000 - 2200°C at a rate of less than 20°C/min and keeping the temperature for at least 2h - 4h;
The conditions of cooling after vacuum sintering further comprise: under a vacuity of no less than 10⁻³Pa, decreasing the temperature of the sintered porous body to 800°C in a manner of decreasing the cooling rate by stages at a rate of no higher than 25 °C/min and no less that 10 °C/min by stages, and keeping the temperature for 30 min - 90 min per stage, and then decreasing to room temperature with the cooling of the furnace;
The conditions of vacuum annealing further comprise: under a vacuity of 10⁻⁴Pa - 10⁻³Pa, increasing the temperature to 800 - 900°C at a rate of 15 °C/min and keeping the temperature for 260 - 320 min, and then decreasing the temperature to 400°C at a rate of 3 °C/min and keeping the temperature for 120 - 300 min, and then cooling to room temperature at a rate of 18 - 23 °C/min.

Based on the means described above, the further characteristics of the present invention are: a vacuity of the vacuum drying is 10⁻² - 1Pa, the drying temperature is 60-100°C, and the drying time is 4 - 8 hours; the conditions of degreasing process further comprise: increasing the temperature to 600-800°C by stages, specifically introducing pure argon (99.9999% of purity) to form the protective atmosphere, increasing the temperature from room temperature to 400°C at a rate of 1 - 5 °C/min and keeping the temperature for 30 - 60 min, then increasing the temperature from 400°C to 600 - 800°C at a rate of 0.5 - 1.5 °C/min and keeping the temperature for 60 - 120 min; the conditions of vacuum sintering further comprise: increasing the temperature from room temperature to 1200 - 1250°C at a rate of 10 - 15 °C/min and keeping the temperature for 30 - 60 min under a vacuity of 10⁻⁴ Pa - 10⁻³Pa; then increasing the temperature to 1500°C at a rate of 10 - 20 °C/min and keeping the temperature for 30 - 60 minutes under a vacuity of 10⁻⁴Pa - 10⁻³Pa, and then increasing the temperature to 2000 - 2200°C at a rate of 6 - 20 °C/min and keeping the temperature for 120 - 240 min under a vacuity of 10⁻⁴Pa - 10⁻³Pa; the conditions of cooling after vacuum sintering further comprise: under a vacuity of 10^{-a}Pa - 10⁻³Pa, decreasing the temperature to 1500 - 1600°C at a rate of 10 - 20 °C/min and keeping the temperature for 30 - 60 min; decreasing the temperature to 1200 - 1250°C at a rate of 12 - 20 °C/min and keeping the temperature for 60 - 90 min; then decreasing the temperature to 800°C at a rate of 10 - 20 °C/min, and then cooling down with the cooling of the furnace; the conditions of vacuum annealing is: increasing the temperature to 800 - 900°C at a rate of 15 °C/min and keeping the temperature for 260 - 320 min under 10⁻⁴ - 10⁻³ Pa of vacuity, then decreasing the temperature to 400°C at a rate of 3 °C/min and keeping the temperature for 120 - 300 min, and then cooling to room temperature at a rate of 18 - 23 °C/min.

Wherein, vacuum drying, degreasing and so on are attributed to reducing the impurities in the porous tantalum, and improving the biocompatibility, bio-safety and mechanical properties. The optimization of the organic foam body overcomes the difficulty of collapse of the foam skeleton during sintering. The optimization of the conditions of the sintering and annealing processes is further favorable to improving the mechanical properties, such as ductility, of the porous tantalum.

### Detailed Description of the Preferred Embodiments

The present invention is more specifically described through the following examples. It should be noted that the scope of protection is defined by the appended claims.

### Example 1:

12.2g of polyvinyl alcohol was weighed and put in a container with 240ml of distilled water, and then the container was put on a hotplate. Polyvinyl alcohol and water are heated and agitated to form an aqueous solution of polyvinyl alcohol. 60g of tantalum powder with a diameter of less than 43µm and an oxygen content of less than 0.1% was weighed by a 200g balance and added to 50ml cooled aqueous solution of polyvinyl alcohol. The tantalum powder and the aqueous solution of polyvinyl alcohol were mixed and agitated well to form a tantalum slurry. The tantalum slurry is filled into a 10×10×30mm porous polyurethane foam body (which has an average pore diameter of 0.48mm, a density of 0.025 g/cm³ and a hardness of 50°) by pressurization with vibration under conditions of 55 times/min and 0.1MPa. Then, the polyurethane foam body filled with the tantalum slurry was dried in a vacuum drier at 60°C for 8 hours under a vacuity of 1Pa. Degreasing process: degreasing at 600°C and keeping the temperature for 120 minutes under a vacuity of lower than 10⁻⁴Pa. Vacuum sintering: sintering is performed in a vacuum furnace at 2000°C and keeping the temperature for 2 hours under a vacuity of 10⁻⁴Pa, and argon was introduced as a protective gas during sintering. The product was cleaned out of the dust and dirt on the surface after the product was took out, and then treated with cooling and thermal treatments. The thermal treatment is increasing the temperature to 800 - 900°C at a rate of 12 °C/min and keeping the temperature for 270 min under a vacuity of 10⁻⁴Pa - 10⁻³Pa, then decreasing the temperature to 400°C at a rate of 4 °C/min and keeping the temperature for 170 min. After that, the product was cooled down to room temperature with the cooling of the furnace, and porous tantalum finished product was obtained.

The density, porosity, pore diameter and other mechanical properties of the obtained porous tantalum finished product were tested by standard test methods such as GB/T5163-2006, GB/T5249-1985, GB/T6886-2001 and the like. The porous tantalum has less than 0.5% of impurities. The pores of the porous tantalum are well-distributed and interconnected. The porous tantalum has a density of 6.7 g/cm³, a porosity of higher than 66%, an average pore diameter of 405µm, an elastic modulus of 4.8GPa, a bending strength of 95MPa, a compressive strength of 72MPa of and a percentage elongation of 10.8%.

### Example 2:

10g of polyvinyl alcohol was weighed and put in a container with 200ml of distilled water, and then the container was put on a hotplate. Polyvinyl alcohol and water are heated and agitated to form an aqueous solution of polyvinyl alcohol. 40g of tantalum powder with a diameter of less than 43µm and an oxygen content of less than 0.1% was weighed by a 200g balance and added to 32ml of the aqueous solution of polyvinyl alcohol. The tantalum powder and the aqueous solution of polyvinyl alcohol were mixed and agitated well to form a tantalum slurry. The tantalum slurry was filled into a 10×10×25mm porous polyurethane foam body (which has an average pore diameter of 0.56mm, a density of 0.030 g/cm³ and a hardness of 60°) by pressurization with vibration under conditions of 60 times/min and 0.08MPa. Then, the polyurethane foam body filled with the tantalum slurry was dried in a vacuum drier at 100°C for 4 hours under a vacuity of 10⁻²Pa. Degreasing process: degreasing at 800°C and keeping the temperature for 120 minutes under a vacuity of 10⁻⁴Pa. Vacuum sintering: sintering is performed in a vacuum furnace at 2100°C and keeping the temperature for 4 hours under a vacuity of 10⁻⁴Pa, and argon was introduced as a protective gas during sintering. The product was cleaned out of the dust and dirt on the surface after the product was took out, and then treated with cooling and thermal treatments. The thermal treatment is increasing the temperature to 800 - 900°C at a rate of 15 °C/min and keeping the temperature for 320 min under a vacuity of 10⁻³Pa, then decreasing the temperature to 400°C at a rate of 3 °C/min and keeping the temperature for 300 min. After that the product was cooled down to room temperature at a rate of 19 °C/min, and porous tantalum finished product was obtained.

The density, porosity, pore diameter and other mechanical properties of the obtained porous tantalum finished product were tested by standard test methods such as GB/T5163-2006, GB/T5249-1985, GB/T6886-2001 and the like. The porous tantalum has less than 0.5% of impurities. The pores of the porous tantalum are well-distributed and interconnected. The porous tantalum has a density of 5.05 g/cm³, a porosity of 58%, an average pore diameter of 330µm, an elastic modulus of 6.0GPa, a bending strength of 93MPa, a compressive strength of 74MPa and a percentage elongation of 11.5%.

### Example 3:

11g of polyvinyl alcohol was weighed and put in a container with 220ml of distilled water, and then the container was put on a hotplate. Polyvinyl alcohol and water are heated and agitated to form an aqueous solution of polyvinyl alcohol. 45g of tantalum powder with a diameter of less than 43µm and an oxygen content of less than 0.1% was weighed by a 200g balance and added to 36ml of the aqueous solution of polyvinyl alcohol. The tantalum powder and the aqueous solution of polyvinyl alcohol were mixed and agitated well to form a tantalum slurry. The tantalum slurry was filled into a 8×8×25mm porous polyurethane foam body (which has an average pore diameter of 0.70mm, a density of 0.035 g/cm³ and a hardness of 70°) by pressurization with vibration under conditions of 80 times/min and 0.2MPa. Then, the polyurethane foam body filled with the tantalum slurry was dried in a vacuum drier at 80°C for 6 hours under a vacuity of 10⁻¹Pa. Degreasing process: degreasing at 700°C and keeping the temperature for 90 minutes under a vacuity of 10⁻³Pa. Vacuum sintering: sintering is performed in vacuum furnace at 2200°C and keeping the temperature for 2.5 hours under a vacuity of 10⁻³Pa, and argon was introduced as a protective gas during sintering. The product was cooled down and took out, then was cleaned out of the dust and dirt on the surface, and then treated with cooling and thermal treatments. The thermal treatment is increasing the temperature to 800 - 900°C at a rate of 17 °C/min and keeping the temperature for 250 min under a vacuity 10⁻³Pa, then decreasing the temperature to 400°C at a rate of 6 °C/min and keeping the temperature for 122 min. After that, the product was cooled down to room temperature at a rate of 18 °C/min, and porous tantalum finished product was obtained.

The density, porosity, pore diameter and other mechanical properties of the obtained porous tantalum finished product were tested by standard test methods such as GB/T5163-2006, GB/T5249-1985, GB/T6886-2001 and the like. The porous tantalum has less than 0.5% of impurities. The pores of the porous tantalum are well-distributed and interconnected. The porous tantalum has a density of 7.5 g/cm³, a porosity of 55% of, an average pore diameter of 130µm, an elastic modulus of 5.2GPa, a bending strength of 106MPa, a compressive strength of 72MPa and a percentage elongation of 10.4%.

| Example | Diameter of tantalum powder (µm)/Oxygen content (%) | Weight concentration of aqueous solution of polyvinyl alcohol | Tantalum powder (weight proportion) : aqueous solution of polyvinyl alcohol (weight proportion) | Ration of weight proportion of the tantalum powder to aqueous solution of polyvinyl alcohol sprayed on surface of the polyurethane foam | Pore diameter of polyureth1ane foam (mm) | Density of polyurethane foam (g/cm³) | Hardness of polyurethane foam (°) |
|---|---|---|---|---|---|---|---|
| 6 | <38/0.1% | 2.8% | 6:1 | 6:1 | 0.80 | 0.025 | 50 |
| 7 | <40/0.1% | 8% | 7.5:1 | 5.5:1 | 0.68 | 0.035 | 55 |
| 8 | <43/0.1% | 6% | 7:1 | 4:1 | 0.70 | 0.030 | 77 |
| 9 | <39/0.1% | 3.4% | 8.8:1 | 7:1 | 0.50 | 0.027 | 66 |

| Example | Vacuity of drying (Pa) / Temperature (°C) / Time (h) | Atmosphere of degreasing (Pa) / Temperature (°C) / Time (min) | Atmosphere of sintering (Pa) / Temperature (°C) / Time (min) | Atmosphere of annealing (Pa) / The rate of increasing or decreasing the temperature (°C/min) / Temperature (°C) /Time of keeping the temperature (min) |
|---|---|---|---|---|
| 6 | 1/75/5.5 | increasing from room temperature to 400 °C at a rate of 1 °C/min and keeping the temperature for 60 min / increasing from 400°C to 600°C at a rate of 0.5 °C/min and keeping the temperature for 120 min | increasing from room temperature to 1200°C at a rate of 10 °C/min and keeping the temperature for 60 min under a vacuity of 10⁻⁴ Pa; increasing to 1250°C at a rate of 11 °C/min and keeping the temperature for 60 min; increasing to 2030°C at a rate of 6 °C/min and keeping the temperature for 240 min under a vacuity of 10⁻³Pa; under a vacuity of 10⁻⁴Pa - 10⁻³Pa ; decreasing to 1520°C at a rate of 11 °C/min and keeping the temperature for 60 min; decreasing to 1200°C at a rate of 13 °C/min and keeping the temperature for 90min; decreasing to 800°C at a rate of 13 °C/min, and cooling with the cooling of the furnace | increasing to 800 - 900°C at a rate of 16 °C/min and keeping the temperature for 360 min under a vacuity of 10⁻⁴ Pa; then decreasing to 400°C at a rate of 2.5 °C/min and keeping the temperature for 150 min, and then cooling down to room temperature with the cooling of the furnace |
| 7 | 1/65/6.5 | increasing from room temperature to 400°C at a rate of 1.5 °C/min and keeping the temperature for 58 min / increasing from 400°C to 650°C at a rate of 0.6 °C/min and keeping the temperature for 110 min | increasing from room temperature to 1210°C at a rate of 11 °C/min and keeping the temperature for 58 min under a vacuity of 1 0⁻⁴Pa; increasing to 1270°C at a rate of 12 °C/min and keeping the temperature for 55 min; increasing to 2050°C at a rate of 8 °C/min and keeping the temperature for 220 min under a vacuity of 10⁻³Pa; under a vacuity of 10⁻⁴Pa10⁻³Pa; decreasing to 1530°C at a rate of 12 °C/min and keeping the temperature for 55 min; decreasing to 1210°C at a rate of 14 °C/min and keeping the temperature for 85 min; decreasing to 800°C at a rate of 14 °C/min and cooling with the cooling of the furnace | increasing to 800 - 900°C at a rate of 18 °C/min and keeping the temperature for 430 min under a vacuity of 10⁻⁴ Pa; then decreasing to 400°C at a rate of 5 °C/min and keeping the temperature for 268 min, and then cooling down to room temperature with the cooling of the furnace |
| 8 | 1/45/7.5 | increasing from room temperature to 400°C at a rate of 2 °C/min and keeping the temperature for 56 min / increasing from 400°C to 680°C at a rate of 0.7°C/min and keeping the temperature for 100 min | increasing from room temperature to 1220°C at a rate of 12 °C/min and keeping the temperature for 55 min under a vacuity of 10⁻⁴ Pa; increasing to 1300°C at a rate of 13 °C/min and keeping the temperature for 50 min; increasing to 2100°C at a rate of 10 °C/min and keeping the temperature for 200 min under a vacuity of 10⁻³Pa; under a vacuity of 10⁻⁴Pa - 10⁻³Pa; decreasing to 1540°C at a rate of 13 °C/min and keeping the temperature for 50 min; decreasing to 1220°C at a rate of 15 °C/min and keeping the temperature for 80 min; decreasing to 800°C at a rate of 15 °C/min and 1 cooling with the cooling of the furnace | increasing to 800 - 900°C at a rate of 12 °C/min and keeping the temperature for 410 min under a vacuity of 10⁻⁴Pa - 10⁻³Pa; then decreasing to 400°C at a rate of 3.5 °C/min and keeping the temperature for 267 min, and hen cooling down to room temperature with the cooling of the furnace |
| 9 | 1/55/7 | increasing from room temperature to 400°C at a rate of 2.5 °C/min and keeping the temperature for 55 min / increasing from 400°C to 700°C at a rate of 0.8 °C/min and keeping the temperature for 90 min | increasing from room temperature to 1230°C at a rate of 13 °C/min and keeping the temperature for 50 min under a vacuity of 10⁻⁴Pa; increasing to 1350°C at a rate of 14 °C/min and keeping the temperature for 45 min; increasing to 2150°C at a rate of 12 °C/min and keeping the temperature for 180 min under a vacuity of 10⁻³Pa; under a vacuity of 10⁻⁴Pa - 10⁻³Pa; decreasing to 1550°C at a rate of 14 °C/min and keeping the temperature for 45 min; decreasing to 1230°C at a rate of 16 °C/min and keeping the temperature for 75 min; decreasing to 800°C at a rate of 16 °C/min and cooling with cooling of the furnace | increasing to 800 - 900°C at a rate of 14 °C/min and keeping the temperature for 400 min under a vacuity of 10⁻³Pa; then decreasing to 400°C at a rate of 2 °C/min and keeping the temperature for 134 min, and then cooling down to room temperature with the cooling of the furnace |

Prepared porous tantalum finished products are tested by the methods described above:

| Example | 6 | 7 | 8 | 9 |
|---|---|---|---|---|
| Density (g/cm³) | 6.4 | 7.3 | 8.0 | 5.8 |
| Porosity (%) | 52 | 70 | 64 | 59 |
| Pore diameter (µm) | 150 | 456 | 389 | 290 |
| Elastic modulus (GPa) | 4.6 | 6.0 | 5.4 | 5.0 |
| Bending strength (MPa) | 110 | 100 | 90 | 80 |
| Compressive strength (MPa) | 80 | 76 | 70 | 73 |
| Percentage elongation (%) | 10.0 | 11.5 | 10.6 | 11.0 |

| | | | | |
|---|---|---|---|---|
| | | | the temperature for 80 min; decreasing to 800°C at a rate of 15 °C/min and 1 cooling with the cooling of the furnace | |
| 9 | 1/55/7 | increasing from room temperature to 400°C at a rate of 2.5 °C/min and keeping the temperature for 55 min / increasing from 400°C to 700°C at a rate of 0.8 °C/min and keeping the temperature for 90 min | increasing from room temperature to 1230°C at a rate of 13 °C/min and keeping the temperature for 50 min under a vacuity of 10⁻⁴Pa; increasing to 1350°C at a rate of 14 °C/min and keeping the temperature for 45 min; increasing to 2150°C at a rate of 12 °C/min and keeping the temperature for 180 min under a vacuity of 10⁻³Pa; under a vacuity of 10⁻⁴Pa - 10⁻³Pa; decreasing to 1550°C at a rate of 14 °C/min and keeping the temperature for 45 min; decreasing to 1230°C at a rate of 16 °C/min and keeping the temperature for 75 min; decreasing to 800°C at a rate of 16 °C/min and cooling with cooling of the furnace | increasing to 800 - 900°C at a rate of 14 °C/min and keeping the temperature for 400 min under a vacuity of 10⁻³Pa; then decreasing to 400°C at a rate of 2 °C/min and keeping the temperature for 134 min, and then cooling down to room temperature with the cooling of the furnace |

Prepared porous tantalum finished products are tested by the methods described above:

| Example | 6 | 7 | 8 | 9 |
|---|---|---|---|---|
| Density (g/cm³) | 6.4 | 7.3 | 8.0 | 5.8 |
| Porosity (%) | 52 | 70 | 64 | 59 |
| Pore diameter (µm) | 150 | 456 | 389 | 290 |
| Elastic modulus (GPa) | 4.6 | 6.0 | 5.4 | 5.0 |
| Bending strength (MPa) | 110 | 100 | 90 | 80 |
| Compressive strength (MPa) | 80 | 76 | 70 | 73 |
| Percentage elongation (%) | 10.0 | 11.5 | 10.6 | 11.0 |

**Table 3 The mechanical properties of the porous tantalum prepared in Example 7-13**

| Example | 6 | 7 | 8 | 9 |
|---|---|---|---|---|
| Density (g/cm³) | 6.4 | 7.3 | 8.0 | 5.8 |
| Porosity (%) | 52 | 70 | 64 | 59 |
| Pore diameter (µm) | 150 | 456 | 389 | 290 |
| Elastic modulus (GPa) | 4.6 | 6.0 | 5.4 | 5.0 |
| Yield strength (MPa) | 110 | 100 | 90 | 80 |
| Compressive strength (MPa) | 80 | 76 | 70 | 73 |
| Percentage elongation (%) | 10.0 | 11.5 | 10.6 | 11.0 |
| Example | 6 | 7 | 8 | 9 |
| Density (g/cm³) | 6.4 | 7.3 | 8.0 | 5.8 |
| Porosity (%) | 52 | 70 | 64 | 59 |
| Pore diameter (µm) | 150 | 456 | 389 | 290 |
| Elastic modulus (GPa) | 4.6 | 6.0 | 5.4 | 5.0 |

## Claims

1. A preparation method for medical porous tantalum implant material, comprising: mixing an aqueous solution of polyvinyl alcohol and a tantalum powder to form a slurry, injecting the slurry into an organic foam body using pressurization with vibration, and going through steps of drying, degreasing, sintering, cooling and thermal treatment to obtain medical porous tantalum material; wherein a mass concentration of the aqueous solution of polyvinyl alcohol is 2 - 8%, a frequency of the vibration is 20 - 80 times/min; the thermal treatment refers to: under a vacuity of 10⁻⁴Pa -10⁻³Pa, increasing a temperature to 800 - 900°C at a rate of 10 - 20 °C/min and keeping the temperature for 240 - 480 minutes, then decreasing the temperature to 400°C at a rate of 2 - 5 °C/min and keeping the temperature for 120 - 300 minutes, then cooling down to room temperature at a rate of 18-23 °C/min.

2. The method according to claim 1, wherein the mass percent of the aqueous solution of polyvinyl alcohol is 4 - 5%, and the ratio of weight proportion of the tantalum powder to weight proportion of the aqueous solution of polyvinyl alcohol is 6 - 9 : 1.

3. The method according to claims 1 or 2, wherein the tantalum powder slurry is filled into the organic foam body through pressurization with vibration, then the aqueous solution of polyvinyl alcohol is sprayed uniformly on the surface of the organic foam body, and the ratio of weight proportion of the sprayed aqueous solution of polyvinyl alcohol to weight proportion of the tantalum powder is 1 : 6.

4. The method according to claim 2, wherein the tantalum powder slurry is filled into the organic foam body through pressurization with vibration, then the aqueous solution of polyvinyl alcohol is sprayed uniformly on the surface of the organic foam body, and the ratio of weight proportion of the sprayed aqueous solution of polyvinyl alcohol to weight proportion of the tantalum powder is 1 : 6.

5. The method according to claim 1, wherein a vacuity during the step of drying is kept at 10⁻² - 1Pa, and degreasing process of removing the polyvinyl alcohol and the organic foam body is conducted under a protective atmosphere, such as under a vacuity of 10⁻⁴ - 10⁻³ Pa, and at a temperature of 400 - 800°C; then a porous sintered body is obtained by vacuum sintering under a vacuity of no less than 10⁻⁴ - 10⁻³ Pa at 2000 - 2200°C and keeping the temperature for 1 - 5 hours; at last, vacuum annealing is conducted;
the conditions of degreasing process further comprise: increasing the temperature to 400 - 800°C at a rate of 0.5 - 5 °C/min, introducing argon to form the protective atmosphere and keeping the temperature for 30 min - 120 min;
the conditions of vacuum sintering further comprise: under a vacuity of no less than 10⁻³ Pa, increasing the temperature from room temperature to 1200°C - 1500°C at a rate of no higher than 10 - 20 °C/min and keeping the temperature for 1 h - 2 h; and then increasing the temperature to 2000 - 2200°C at a rate of less than 20 °C/min and keeping the temperature for at least 2-4 hours; the conditions of cooling after the vacuum sintering further comprise: under a vacuity of no less than 10⁻³Pa, decreasing the temperature of the sintered porous body to 800°C by stages in a manner of decreasing the cooling rate by stages at a rate of no higher than 25 °C/min and no less than 10 °C/min, in each stage keeping the temperature for 30 min - 90 min, and then cooling down to room temperature with the cooling of the furnace; and
the steps of thermal treatment is: under a vacuity of 10⁻⁴Pa - 10⁻³Pa, increasing the temperature to 800 - 900°C at a rate of 15 °C/min and keeping the temperature for 260 - 320 min, and then decreasing the temperature to 400°C at a rate of 3 °C/min and keeping the temperature for 120 - 300 min, and then cooling down to room temperature at a rate of 18 - 23 °C/min.

6. The method according to claim 4, wherein the conditions of degreasing process further comprise: increasing the temperature to 400 - 800°C by stages at a rate of 0.5 °C/min - 5 °C/min, introducing argon to form the protective atmosphere and keeping the temperature for 30 min. - 120 min;
the conditions of vacuum sintering further comprise: under a vacuity of no less than 10⁻³Pa, increasing the temperature from room temperature to 1200°C - 1500°C at a rate of no higher than 10 - 20 °C/min and keeping the temperature for 1 h - 2 h, and then increasing the temperature to 2000 - 2200°C at a rate of less than 20 °C/min and keeping the temperature for at least 2 h - 4 h;
the conditions of cooling after vacuum sintering further comprise: under a vacuity of no less than 10⁻³Pa, decreasing the temperature of the sintered porous body to 800°C by stages in a manner of decreasing the cooling rate by stages at a rate of no higher than 25 °C/min and no less than 10 °C/min, in each stage keeping the temperature for 30 min - 90 min, and then cooling down to room temperature with the cooling of the furnace; and
the conditions of the vacuum annealing further comprise that the thermal treatment is under a vacuity of 10⁻⁴Pa - 10⁻³Pa, increasing the temperature to 800 - 900°C at a rate of 15 °C/min and keeping the temperature for 260 - 320 min, and then decreasing the temperature to 400°C at a rate of 3 °C/min and keeping the temperature for 120 - 300 min, and then cooling down to room temperature at a rate of 18 - 23 °C/min.

7. The method according to claims 1, wherein the vacuum drying is under a vacuity of 10⁻² - 1Pa, at the temperature of 60 - 100°C, and with a drying time of 4 - 8 hours; the conditions of degreasing process further comprise: increasing the temperature to 600 - 800°C by stages, then introducing pure argon (99.9999%) to form the protective atmosphere, increasing the temperature from room temperature to 400°C at a rate of 1 - 5 °C/min and keeping the temperature for 30 - 60 min, and then increasing the temperature from 400°C to 600 - 800°C at a rate of 0.5 - 1.5 °C/min and keeping the temperature for 60 - 120 min; the conditions of vacuum sintering further comprise: increasing the temperature from room temperature to 1200 - 1250°C at a rate of 10 - 15 °C/min and keeping the temperature for 30 - 60 min under a vacuity of 10⁻⁴Pa - 10⁻³Pa; then increasing the temperature to 1500°C at a rate of 10 - 20 °C/min and keeping the temperature for 30 - 60 min under a vacuity of 10⁻⁴Pa- 10⁻³Pa, and then increasing the temperature to 2000 - 2200°C at a rate of 6 - 20 °C/min and keeping the temperature for 120 - 240 min under a vacuity of 10⁻⁴Pa - 10⁻³Pa; the conditions of cooling after vacuum sintering further comprise: under a vacuity of 10⁻⁴Pa - 10⁻³Pa; decreasing the temperature to 1500 - 1600°C at a rate of 10 - 20 °C/min and keeping the temperature for 30 - 60 min; then decreasing the temperature to 1200 - 1250°C at a rate of 12 - 20 °C/min and keeping the temperature for 60 - 90 minutes; and then decreasing the temperature to 800°C at a rate of 10 - 20 °C/min, and cooling down with the cooling of the furnace; the conditions of vacuum annealing further comprise that the thermal treatment is under a vacuity of 10⁻⁴Pa - 10⁻³Pa, increasing the temperature to 800 - 900°C at a rate of 15 °C/min and keeping the temperature for 260 - 320 min, then decreasing the temperature to 400°C at a rate of 3 °C/min and keeping the temperature for 120 - 300 min, and then cooling down to room temperature at a rate of 18 - 23 °C/min.

8. The method according to claim 4, wherein the vacuum drying is under a vacuity of 10⁻² - 1Pa, at the temperature of 60-100°C, with a drying time of 4-8 hours; the conditions of degreasing process further comprise: increasing the temperature to 600 - 800°C by stages, then introducing pure argon (99.9999%) to form the protective atmosphere, increasing the temperature from room temperature to 400°C at a rate of 1 - 5 °C/min and keeping the temperature for 30 - 60 min, and then increasing the temperature from 400°C to 600 - 800°C at a rate of 0.5 - 1.5 °C/min and keeping the temperature for 60 - 120 min; the conditions of vacuum sintering further comprise: increasing the temperature to 1200 - 1250°C at a rate of 10 - 15 °C/min and keeping the temperature for 30 - 60 min under a vacuity of 10⁻⁴Pa - 10⁻³Pa; then increasing the temperature to 1500°C at a rate of 10 - 20 °C/min and keeping the temperature for 30 - 60 min under a vacuity of 10⁻⁴Pa - 10⁻³Pa, and then increasing the temperature to 2000 - 2200°C at a rate of 6 - 20 °C/min and keeping the temperature for 120 - 240 min under a vacuity of 10⁻⁴Pa - 10⁻³Pa; the conditions of cooling after vacuum sintering further comprise: under a vacuity of 10⁻⁴ - 10⁻³ Pa; decreasing the temperature to 1500 - 1600°C at a rate of 10 - 20 °C/min and keeping the temperature for 30 - 60 min; then decreasing the temperature to 1200 - 1250 °C at a rate of 12 - 20°C/min and keeping the temperature for 60 - 90 minutes; and then decreasing the temperature to 800°C at a rate of 10 - 20 °C/min, and cooling down with the cooling of the furnace; and the conditions of vacuum annealing further comprise that the thermal treatment is under a vacuity of 10⁻⁴Pa - 10⁻³Pa, increasing the temperature to 800 - 900°C at a rate of 15 °C/min and keeping the temperature for 260 - 320 min, then decreasing the temperature to 400°C at a rate of 3 °C/min and keeping the temperature for 120 - 300 min, and then cooling down to room temperature at a rate of 18 - 23 °C/min.

## Patentansprüche

1. Verfahren zur Herstellung eines medizinischen porösen Tantalimplantatmaterials, umfassend: Mischen einer wässrigen Lösung aus Polyvinylalkohol und Tantalpulver zur Bildung einer Aufschlämmung, Injizieren der Aufschlämmung in einen organischen Schaumkörper unter Verwendung von Druckbeaufschlagung mit Vibration und Durchlaufen von Schritten zum Trocknen, Entfetten, Sintern, Kühlen und Wärmebehandlung, um medizinisches poröses Tantalmaterial zu erhalten; wobei eine Massekonzentration der wässrigen Lösung aus Polyvinylalkohol 2 - 8 % ist, eine Frequenz der Vibration 20 - 80 mal/min ist; wobei sich die Wärmebehandlung bezieht auf: unter einem Vakuumgrad von 10⁻⁴ Pa - 10⁻³ Pa, Erhöhen einer Temperatur auf 800 - 900 °C bei einer Rate von 10 - 20 °C/min und Halten der Temperatur für 240 - 480 Minuten, dann Senken der Temperatur auf 400 °C bei einer Rate von 2 - 5 °C/min und Halten der Temperatur für 120 - 300 Minuten, dann Abkühlen auf Raumtemperatur bei einer Rate von 18 - 23 °C/min.

2. Verfahren nach Anspruch 1, wobei die Masseprozent der wässrigen Lösung aus Polyvinylalkohol 4 - 5 % sind und das Verhältnis des Gewichtsanteils des Tantalpulvers zu Gewichtsanteil der wässrigen Lösung aus Polyvinylalkohol 6 - 9 : 1 ist.

3. Verfahren nach Anspruch 1 oder 2, wobei die Tantalpulveraufschlämmung in den organischen Schaumkörper durch Druckbeaufschlagung mit Vibration gefüllt wird, dann die wässrige Lösung aus Polyvinylalkohol gleichförmig auf die Oberfläche des organischen Schaumkörpers gesprüht wird und das Verhältnis von Gewichtsanteil der gesprühten wässrigen Lösung aus Polyvinylalkohol zu Gewichtsanteil des Tantalpulvers 1 : 6 ist.

4. Verfahren nach Anspruch 2, wobei die Tantalpulveraufschlämmung in den organischen Schaumkörper durch Druckbeaufschlagung mit Vibration gefüllt wird, dann die wässrige Lösung aus Polyvinylalkohol gleichförmig auf die Oberfläche des organischen Schaumkörpers gesprüht wird und das Verhältnis von Gewichtsanteil der gesprühten wässrigen Lösung aus Polyvinylalkohol zu Gewichtsanteil des Tantalpulvers 1 : 6 ist.

5. Verfahren nach Anspruch 1, wobei ein Vakuumgrad während des Trocknungsschritts bei 10⁻² - 1 Pa gehalten wird und Entfettungsprozess zum Entfernen des Polyvinylalkohols und des organischen Schaumkörpers unter einer Schutzatmosphäre, wie unter einem Vakuumgrad von 10⁻⁴ Pa - 10⁻³ Pa, und bei einer Temperatur von 400 - 800 °C durchgeführt wird; dann ein poröser gesinterter Körper durch Vakuumsintern unter einem Vakuumgrad von nicht weniger als 10⁻⁴ Pa - 10⁻³ Pa bei 2000 - 2200 °C und Halten der Temperatur für 1 - 5 Stunden erhalten wird; schließlich Vakuumtempern durchgeführt wird;
die Bedingungen des Entfettungsprozesses weiter umfassen: Erhöhen der Temperatur auf 400 - 800 °C bei einer Rate von 0,5 - 5 °C/min, Einleiten von Argon zum Bilden der Schutzatmosphäre und Halten der Temperatur für 30 min - 120 min;
die Bedingungen von Vakuumsintern weiter umfassen: unter einem Vakuumgrad von nicht weniger als 10⁻³ Pa, Erhöhen der Temperatur von Raumtemperatur auf 1200 °C - 1500 °C bei einer Rate von nicht mehr als 10 - 20 °C/min und Halten der Temperatur für 1 Std. - 2 Std.; und dann Erhöhen der Temperatur auf 2000 - 2200 °C bei einer Rate von kleiner als 20 °C/min und Halten der Temperatur für mindestens 2 - 4 Stunden;
die Bedingungen von Kühlen nach dem Vakuumsintern weiter umfassen: unter einem Vakuumgrad von nicht weniger als 10⁻³ Pa, stufenweises Senken der Temperatur des gesinterten porösen Körpers auf 800 °C in einer Weise, dass die Kühlungsrate stufenweise bei einer Rate von nicht mehr als 25 °C/min und nicht weniger als 10 °C/min gesenkt wird, in jeder Stufe die Temperatur 30 min - 90 min gehalten wird, und dann mit Kühlen des Ofens auf Raumtemperatur abgekühlt wird; und
die Schritte einer Wärmebehandlung sind: unter einem Vakuumgrad von 10⁻⁴ Pa - 10⁻³ Pa, Erhöhen der Temperatur auf 800 - 900 °C bei einer Rate von 15 °C/min und Halten der Temperatur für 260 - 320 min und dann Senken der Temperatur auf 400 °C bei einer Rate von 3 °C/min und Halten der Temperatur für 120 - 300 min und dann Abkühlen auf Raumtemperatur bei einer Rate von 18 - 23 °C/min.

6. Verfahren nach Anspruch 4, wobei die Bedingungen des Entfettungsprozesses weiter umfassen: stufenweises Erhöhen der Temperatur auf 400 - 800 °C bei einer Rate von 0,5 °C/min - 5 °C/min, Einleiten von Argen zur Bildung der Schutzatmosphäre und Halten der Temperatur für 30 min - 120 min;
die Bedingungen von Vakuumsintern weiter umfassen: unter einem Vakuumgrad von nicht kleiner als 10⁻³ Pa, Erhöhen der Temperatur von Raumtemperatur auf 1200 °C - 1500 °C bei einer Rate nicht höher als 10 - 20 °C/min und Halten der Temperatur für 1 Std. - 2 Std. und dann Erhöhen der Temperatur auf 2000 - 2200 °C bei einer Rate von kleiner als 20 °C/min und Halten der Temperatur für mindestens 2 Std. - 4 Std.;
die Bedingungen von Kühlen nach Vakuumsintern weiter umfassen: unter einem Vakuumgrad von nicht weniger als 10⁻³ Pa, stufenweises Senken der Temperatur des gesinterten porösen Körpers auf 800 °C in einer Weise, dass die Kühlungsrate stufenweise bei einer Rate von nicht mehr als 25 °C/min und nicht weniger als 10 °C/min gesenkt wird, in jeder Stufe Halten der Temperatur für 30 min - 90 min und dann mit Kühlen des Ofens Abkühlen auf Raumtemperatur; und
die Bedingungen des Vakuumtemperns weiter umfassen, dass die Wärmebehandlung unter einem Vakuumgrad von 10⁻⁴ Pa - 10⁻³ Pa durch Erhöhen der Temperatur auf 800 - 900 °C bei einer Rate von 15 °C/min und Halten der Temperatur für 260 - 320 min und dann Senken der Temperatur auf 400 °C bei einer Rate von 3 °C/min und Halten der Temperatur für 120 - 300 min und dann Abkühlen auf Raumtemperatur bei einer Rate von 18 - 23 °C/min erfolgt.

7. Verfahren nach Anspruch 1, wobei die Vakuumtrocknung unter einem Vakuumgrad von 10⁻² - 1Pa bei der Temperatur von 60 - 100 °C und mit einer Trocknungszeit von 4 - 8 Stunden ist; die Bedingungen des Entfettungsprozesses weiter umfassen: stufenweises Erhöhen der Temperatur auf 600 - 800 °C, dann Einleiten von reinem Argon (99,9999 %) zur Bildung der Schutzatmosphäre, Erhöhen der Temperatur von Raumtemperatur auf 400 °C bei einer Rate von 1 - 5 °C/min und Halten der Temperatur für 30 - 60 min und dann Erhöhen der Temperatur von 400 °C auf 600 - 800 °C bei einer Rate von 0,5 - 1,5 °C/min und Halten der Temperatur für 60 - 120 min; die Bedingungen von Vakuumsintern weiter umfassen: Erhöhen der Temperatur von Raumtemperatur auf 1200 °C - 1250 °C bei einer Rate von 10 - 15 °C/min und Halten der Temperatur für 30 - 60 min unter einem Vakuumgrad von 10⁻⁴ Pa - 10⁻³ Pa; dann Erhöhen der Temperatur auf 1500 °C bei einer Rate von 10 - 20 °C/min und Halten der Temperatur für 30 - 60 min unter einem Vakuumgrad von 10⁻⁴ Pa - 10⁻³ Pa und dann Erhöhen der Temperatur auf 2000 - 2200 °C bei einer Rate von 6 - 20 °C/min und Halten der Temperatur für 120 - 240 min unter einem Vakuumgrad von 10⁻⁴ Pa - 10⁻³ Pa; die Bedingungen von Kühlen nach dem Vakuumsintern weiter umfassen: unter einem Vakuumgrad von 10⁻⁴ Pa - 10⁻³ Pa; Senken der Temperatur auf 1500 - 1600 °C bei einer Rate von 10 - 20 °C/min und Halten der Temperatur für 30 - 60 min; dann Senken der Temperatur auf 1200 - 1250 °C bei einer Rate von 12 - 20 °C/min und Halten der Temperatur für 60 - 90 Minuten und dann Senken der Temperatur auf 800 °C bei einer Rate von 10 - 20 °C/min und Abkühlen mit der Kühlung des Ofens; die Bedingungen von Vakuumtempern weiter umfassen, dass die Wärmebehandlung, unter einem Vakuumgrad von 10⁻⁴ Pa - 10⁻³ Pa, Erhöhen der Temperatur auf 800 - 900 °C bei einer Rate von 15 °C/min und Halten der Temperatur für 260 - 320 min, dann Senken der Temperatur auf 400 °C bei einer Rate von 3 °C/min und Halten der Temperatur für 120 - 300 min und dann Abkühlen auf Raumtemperatur bei einer Rate von 18 - 23 °C ist.

8. Verfahren nach Anspruch 4 wobei die Vakuumtrocknung unter einem Vakuumgrad von 10⁻² - 1Pa bei der Temperatur von 60 - 100 °C, mit einer Trocknungszeit von 4 - 8 Stunden ist; die Bedingungen des Entfettungsprozesses weiter umfassen: stufenweises Erhöhen der Temperatur auf 600 - 800 °C, dann Einleiten von reinem Argon (99,9999 %) zur Bildung der Schutzatmosphäre, Erhöhen der Temperatur von Raumtemperatur auf 400 °C bei einer Rate von 1 - 5 °C/min und Halten der Temperatur für 30 - 60 min und dann Erhöhen der Temperatur von 400 °C auf 600 - 800 °C bei einer Rate von 0,5 - 1,5 °C/min und Halten der Temperatur für 60 - 120 min; die Bedingungen von Vakuumsintern weiter umfassen: Erhöhen der Temperatur auf 1200 °C - 1250 °C bei einer Rate von 10 - 15 °C/min und Halten der Temperatur für 30 - 60 min unter einem Vakuumgrad von 10⁻⁴ Pa - 10⁻³ Pa; dann Erhöhen der Temperatur auf 1500 °C bei einer Rate von 10 - 20 °C/min und Halten der Temperatur für 30 - 60 min unter einem Vakuumgrad von 10⁻⁴ Pa - 10⁻³ Pa und dann Erhöhen der Temperatur auf 2000 - 2200 °C bei einer Rate von 6 - 20 °C/min und Halten der Temperatur für 120 - 240 min unter einem Vakuumgrad von 10⁻⁴ Pa - 10⁻³ Pa; die Bedingungen von Kühlen nach dem Vakuumsintern weiter umfassen: unter einem Vakuumgrad von 10⁻⁴ Pa - 10⁻³ Pa; Senken der Temperatur auf 1500 - 1600 °C bei einer Rate von 10 - 20 °C/min und Halten der Temperatur für 30 - 60 min; dann Senken der Temperatur auf 1200 - 1250 °C bei einer Rate von 12 - 20 °C/min und Halten der Temperatur für 60 - 90 Minuten; und dann Senken der Temperatur auf 800 °C bei einer Rate von 10 - 20 °C/min und Abkühlen mit der Kühlung des Ofens; die Bedingungen von Vakuumtempern weiter umfassen, dass die Wärmebehandlung, unter einem Vakuumgrad von 10⁻⁴ Pa - 10⁻³ Pa, Erhöhen der Temperatur auf 800 - 900 °C bei einer Rate von 15 °C/min und Halten der Temperatur für 260 - 320 min, dann Senken der Temperatur auf 400 °C bei einer Rate von 3 °C/min und Halten der Temperatur für 120 - 300 min und dann Abkühlen auf Raumtemperatur bei einer Rate von 18 - 23 °C ist.

## Revendications

1. Un procédé de préparation d'un matériau d'implant médical poreux en tantale, comprenant : le fait de mélanger une solution aqueuse d'alcool polyvinylique et une poudre de tantale pour former une suspension, le fait d'injecter la suspension sous la forme d'un corps en mousse organique en utilisant une pressurisation avec vibration, et de passer par des étapes de séchage, de dégraissage, de frittage, de refroidissement et de traitement thermique pour obtenir un matériau médical poreux en tantale ; une concentration massique de la solution aqueuse d'alcool polyvinylique étant de 2 à 8%, une fréquence de vibration étant de 20 à 80 fois/min ; le traitement thermique faisant référence à : sous une dépression de 10⁻⁴Pa à 10⁻³Pa, le fait d'augmenter une température à 800 à 900°C à un taux de 10 à 20°C/min et de maintenir la température pendant 240 à 480 minutes, puis de diminuer la température à 400°C à un taux de 2 à 5°C/min et de maintenir la température pendant 120 à 300 minutes, puis le fait de refroidir à la température ambiante à un taux de 18 à 23°C/min.

2. Le procédé selon la revendication 1, dans lequel le pourcentage en masse de la solution aqueuse d'alcool polyvinylique est de 4 à 5 %, et le rapport de la proportion en poids de la poudre de tantale à la proportion en poids de la solution aqueuse d'alcool polyvinylique est de 6 à 9:1.

3. Le procédé selon la revendication 1 ou la revendication 2, dans lequel la suspension de poudre de tantale est introduite dans le corps en mousse organique par pressurisation avec vibration, puis la solution aqueuse d'alcool polyvinylique est pulvérisée uniformément sur la surface du corps en mousse organique, et le rapport de la proportion pondérale de la solution aqueuse pulvérisée d'alcool polyvinylique à la proportion pondérale de la poudre de tantale est de 1:6.

4. Le procédé selon la revendication 2, dans lequel la suspension de poudre de tantale est introduite dans le corps en mousse organique par pressurisation avec vibration, puis la solution aqueuse d'alcool polyvinylique est pulvérisée uniformément sur la surface du corps en mousse organique, et le rapport de la proportion pondérale de la solution aqueuse pulvérisée d'alcool polyvinylique à la proportion pondérale de la poudre de tantale est de 1:6.

5. Le procédé selon la revendication 1, dans lequel une dépression pendant l'étape de séchage est maintenue entre 10⁻² et 1Pa, et le processus de dégraissage pour éliminer l'alcool polyvinylique et le corps en mousse organique est effectué sous une atmosphère protectrice, comme par exemple sous une dépression de 10⁻⁴ à 10⁻³ Pa, et à une température de 400 à 800°C ; ensuite, un corps fritté poreux est obtenu par frittage sous vide sous une dépression d'au moins 10⁻⁴ à 10⁻³ Pa à 2000 à 2200°C et en maintenant la température pendant 1 à 5 heures ; enfin, un recuit sous vide est effectué ;
les conditions du processus de dégraissage comprennent en outre : le fait d'augmenter la température à 400 à 800°C à un taux de 0,5 à 5°C/min, le fait d'introduire de l'argon pour former l'atmosphère protectrice et de maintenir la température pendant 30 min à 120 min ;
les conditions de frittage sous vide comprennent en outre : sous une dépression d'au moins 10⁻³ Pa, le fait d'augmenter la température depuis la température ambiante à 1200°C à 1500°C à un taux ne dépassant pas 10 à 20°C/min et de maintenir la température pendant 1 h à 2 h ; et ensuite le fait d'augmenter la température à 2000 à 2200°C à un taux inférieur à 20°C/min et de maintenir la température pendant au moins 2 à 4 heures ;
les conditions de refroidissement après le frittage sous vide comprennent en outre : sous une dépression non inférieure à 10⁻³Pa, le fait de réduire la température du corps poreux fritté à 800°C par étapes de manière à diminuer le taux de refroidissement par étapes à un taux non supérieur à 25°C/min et non inférieur à 10°C/min, dans chaque étape le fait de maintenir la température pendant 30 min à 90 min, puis le fait de refroidir à la température ambiante avec le refroidissement du four ; et
les étapes du traitement thermique sont : sous une dépression de 10⁻³Pa à 10⁻³Pa, le fait d'augmenter la température à 800 à 900°C à un taux de 15°C/min et de maintenir la température pendant 260 à 320 min, puis de diminuer la température à 400°C à un taux de 3°C/min et de maintenir la température pendant 120 à 300 min, puis de refroidir à la température ambiante à un taux de 18 à 23°C/min.

6. Le procédé selon la revendication 4, dans lequel les conditions du processus de dégraissage comprennent en outre : le fait d'augmenter la température à 400 à 800°C par étapes à un taux de 0,5°C/min à 5°C/min, d'introduire de l'argon pour former l'atmosphère protectrice et de maintenir la température pendant 30 min à 120 min ;
les conditions de frittage sous vide comprennent en outre : sous une dépression non inférieure à 10⁻³Pa, le fait d'augmenter la température depuis la température ambiante à 1200°C à 1500°C à un taux non supérieur à 10 à 20°C/min et de maintenir la température pendant 1 h à 2 h, puis d'augmenter la température à 2000 à 2200°C à un taux inférieur à 20°C/min et de maintenir la température pendant au moins 2 h à 4 h ;
les conditions de refroidissement après le frittage sous vide comprennent en outre : sous une dépression d'au moins 10⁻³Pa, le fait d'abaisser la température du corps poreux fritté à 800°C par étapes de manière à diminuer le taux de refroidissement par étapes à un taux non supérieur à 25°C/min et non inférieur à 10°C/min, dans chaque étape le fait de maintenir la température pendant 30 min à 90 min, puis le fait de refroidir à la température ambiante avec le refroidissement du four ; et
les conditions du recuit sous vide comprennent en outre le fait que le traitement thermique se fait sous une dépression de 10⁻⁴Pa- 10⁻³Pa, le fait d'augmenter la température à 800 à 900°C à un taux de 15°C/min et de maintenir la température pendant 260 à 320 min, puis le fait de diminuer la température à 400°C à un taux de 3°C/min et de maintenir la température pendant 120 à 300 min, puis le fait de refroidir à la température ambiante à un taux de 18 à 23°C/min.

7. Le procédé selon la revendication 1, dans laquelle le séchage sous vide se fait sous une dépression de 10⁻² à 1Pa, à la température de 60 à 100°C, et avec un temps de séchage de 4 à 8 heures ; les conditions du processus de dégraissage comprennent en outre : le fait d'augmenter la température à 600 à 800°C par étapes, puis le fait d'introduire de l'argon pur (99,9999%) pour former l'atmosphère protectrice, le fait d'augmenter la température de la température ambiante à 400°C à un taux de 1 à 5°C/min et de maintenir la température pendant 30 à 60 min, puis le fait d'augmenter la température de 400°C à 600 à 800°C à un taux de 0,5 à 1,5°C/min et de maintenir la température pendant 60 à 120 min ; les conditions de frittage sous vide comprennent en outre : le fait d'augmenter la température depuis la température ambiante à 1200 à 1250°C à un taux de 10 à 15°C/min et de maintenir la température pendant 30 à 60 min sous une dépression de 10⁻⁴Pa à 10⁻³Pa ; puis d'augmenter la température à 1500°C à un taux de 10 à 20°C/min et de maintenir la température pendant 30 à 60 minutes sous une dépression de 10⁻⁴Pa à 10⁻³Pa, puis d'augmenter la température à 2000 à 2200°C à un taux de 6 à 20°C/min et de maintenir la température pendant 120 à 240 minutes sous une dépression de 10⁻⁴Pa à 10⁻³Pa ; les conditions de refroidissement après frittage sous vide comprennent en outre : sous une dépression de 10⁻⁴Pa à 10⁻³Pa, le fait de diminuer la température à 1500 à 1600°C à un taux de 10 à 20°C/min et de maintenir la température pendant 30 à 60 min ; puis le fait de diminuer la température à 1200 à 1250°C à un taux de 12 à 20°C/min et de maintenir la température pendant 60 à 90 minutes ; puis le fait de diminuer la température à 800°C à un taux de 10 à 20°C/min, et le fait de refroidir avec le refroidissement du four ; les conditions de recuit sous vide comprennent en outre le fait que le traitement thermique se fait sous une dépression de 10⁻⁴Pa à 10⁻³Pa, le fait d'augmenter la température à 800 à 900°C à un taux de 15°C/min et de maintenir la température pendant 260 à 320 min, puis le fait de diminuer la température à 400°C à un taux de 3°C/min et de maintenir la température pendant 120 à 300 min, puis le fait de refroidir à la température ambiante à un taux de 18 à 23°C/min.

8. Le procédé selon la revendication 4, dans lequel le séchage sous vide se fait sous une dépression de 10⁻² à 1Pa, à la température de 60 à 100°C, avec un temps de séchage de 4 à 8 heures ; les conditions du processus de dégraissage comprennent en outre : le fait d'augmenter la température à 600 à 800°C par étapes, puis le fait d'introduire de l'argon pur (99,9999%) pour former l'atmosphère protectrice, le fait d'augmenter la température depuis la température ambiante à 400°C à un taux de 1 à 5°C/min et de maintenir la température pendant 30 à 60 min, puis le fait d'augmenter la température de 400°C à 600 à 800°C à un taux de 0,5 à 1,5°C/min et de maintenir la température pendant 60 à 120 min ; les conditions de frittage sous vide comprennent en outre : le fait d'augmenter la température à 1200 à 1250°C à un taux de 10 à 15°C/min et de maintenir la température pendant 30 à 60 min sous une dépression de 10⁻⁴Pa à 10⁻³Pa ; puis le fait d'augmenter la température à 1500°C à un taux de 10 à 20°C/min et de maintenir la température pendant 30 à 60 minutes sous une dépression de 10⁻⁴Pa à 10⁻³Pa, puis le fait d'augmenter la température à 2000 à 2200°C à un taux de 6 à 20°C/min et de maintenir la température pendant 120 à 240 minutes sous une dépression de 10⁻⁴Pa à 10⁻³Pa ; les conditions de refroidissement après le frittage sous vide comprennent en outre : sous une dépression de 10⁻⁴ à 10⁻³ Pa, le fait de diminuer la température à 1500 à 1600°C à un taux de 10 à 20°C/min et de maintenir la température pendant 30 à 60 min ; puis le fait de diminuer la température à 1200 à 1250°C à un taux de 12 à 20°C/min et de maintenir la température pendant 60 à 90 minutes ; puis le fait de diminuer la température à 800°C à un taux de 10 à 20°C/min, et le fait de refroidir avec le refroidissement du four ; et les conditions de recuit sous vide comprennent en outre le fait que le traitement thermique se fait sous une dépression de 10⁻⁴Pa à 10⁻³Pa, le fait d'augmenter la température à 800 à 900°C à un taux de 15°C/min et de maintenir la température pendant 260 à 320 min, puis le fait de diminuer la température à 400°C à un taux de 3°C/min et de maintenir la température pendant 120 à 300 min, puis le fait de refroidir à la température ambiante à un taux de 18 à 23°C/min.
